Europäisches Patentamt

European Patent Office

Office européen des brevets

(19)

(11) Publication number: **0 172 480**
**A2**

(12) # EUROPEAN PATENT APPLICATION

(21) Application number: **85109783.2**

(22) Date of filing: **03.08.85**

(51) Int. Cl.⁴: **A 61 D 7/00**
**A 61 M 5/14**

(30) Priority: **18.08.84 GB 8421046**

(43) Date of publication of application:
**26.02.86 Bulletin 86/9**

(84) Designated Contracting States:
**BE DE FR GB NL**

(71) Applicant: **BEECHAM GROUP PLC**
**Beecham House Great West Road**
**Brentford Middlesex TW8 9BD(GB)**

(72) Inventor: **Bywater, Robert James**
**1 Shelvers Way**
**Tadworth Surrey(GB)**

(74) Representative: **Hardman, Carol Pauline et al,**
**Beecham Pharmaceuticals Great Burgh Yew Tree**
**Bottom Road**
**Epsom Surrey KT18 5XQ(GB)**

(54) Veterinary device.

(57) An infusion device comprising liquid supply means (1), a delivery tube (4) and a injection means (9), the delivery tube (4) interconnecting said supply means (1) with said injection means (9) and being arranged such that it presents enlarged surface area along a portion (7) of its length whereby said portion (7) acts as heat receiving means.

EP 0 172 480 A2

Croydon Printing Company Ltd.

## VETERINARY DEVICE

This invention relates to a device for quickly delivering a warm veterinary infusion liquid to an animal.

In the treatment of animals such as cows suffering from coli-mastitis and dehydrated diarrhoeic calves it is often necessary to administer rapidly large volumes of liquid by intravenous infusion. To avoid the potentially fatal stress of infusing cold liquid it is usual to warm the liquid before treatment commences. However, typically from 5 to 20 litres of liquid are required and warming such large volumes can result in considerable delay which can also be dangerous to the animal.

The present invention seeks to overcome these problems by providing means by which the infusion liquid may be warmed during passage through the delivery tube.

According to the present there is provided an infusion device comprising a liquid supply means, a delivery tube and an injection means, the delivery tube interconnecting said supply means with said injection means and being arranged such that it presents an enlarged surface area along a portion of its length whereby said portion acts as heat receiving means.

Suitably the liquid supply means comprises a reservoir for infusion liquid and dose control means, for example, a drip chamber and control valve. The injection means suitably comprises a hollow needle or a cannula, arranged at the end of the delivery tube remote from the reservoir.

Suitably the delivery tube comprises a plastics material, for example polyethylene, polypropylene or polyvinylchloride and is connected to other components of the device via Luer fittings or rubber connectors.

Suitably the heat receiving portion of the delivery tube consists of a coil. The coil may be of metal or plastics material; conveniently it is of the same material as, and is formed integrally with, the delivery tube. Most preferably the delivery tube including heat receiving portion thereof are formed of polyvinylchloride tubing of the type conventionally used in infusion apparatus.

The device of the invention is used as a conventional 'giving set' except that the heat receiving portion of the delivery tube is immersed in hot water to warm the infusion liquid during transit through the delivery tube.

Usually the liquid flows under gravity and the flow rate is observed in the drip chamber and adjusted using the tap, but for convenience, or if very high flow rates are required, a peristaltic pump may be used. Suitably flow rates of up to 80 ml min$^{-1}$ are employed.

The device may be used to administer conventional intravenous infusion liquids including plasma expanding solutions and intravenous rehydration solutions.

The invention will now be described by way of example with reference to the accompanying diagrammatic drawings in which:-

Fig. 1 shows, in schematic form, a device of the invention, and
Fig. 2 shows an alternative delivery tube.

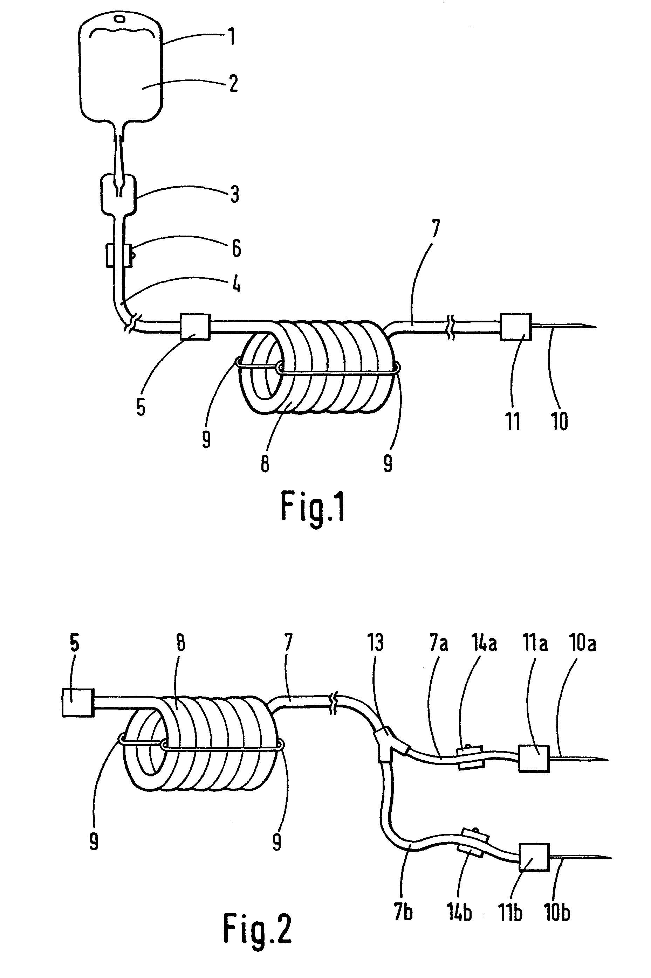

The device shown in Fig. 1 comprises a reservoir 1 (containing infusion liquid 2) connected to a drip chamber 3. The drip chamber 3 is connected to a delivery tube 4. A tap 6 is arranged to control fluid flow between the drip chamber 2 and the delivery tube 4.

The delivery tube 4 is connected by way of a Luer fitting 5 to a second delivery tube 7 having a coil 8 provided along its length. Suitably both delivery tubes 4 and 7 are formed from plastic tubing having an inside diameter of 3 mm and an outside diameter of 5 mm. The turns of the coiled 8 are held together by wires 9. In alternative embodiments the turns of the coil 8 are held together by heat sealing or welding together the successive turns or by enclosing the coil in a shrink-wrap membrane.

A needle 10 is connected to the free end of the delivery tube 7 by a luer fitting 11.

In use the needle 10 is inserted into the mammary or jugular vein of the animal to be treated, the coil 8 is immersed in hot water contained in a bucket or other container and the infusion liquid 2 is allowed to flow under gravity or with mechanical assistance, for example by means of a peristaltic pump (not shown) through the apparatus. The flow rate is observed in the drip chamber 3 and adjusted by means of the tap 6. When one reservoir 1 is expended, it may be removed and a new reservoir can then be attached to the drip chamber 3 to continue the infusion.

In alternative embodiments the needle 10 may be
replaced by a cannula.

An alternative embodiment of the delivery tube 7 is
shown in Figure 2.  In this embodiment a Y-piece 13 is
inserted in the tube 7, after the coil 8 to provide two
branch tubes 7a, 7b, each of which is connected to a
needle 10a, 10b by way of Luer fittings 11a, 11b
respectively.  A tap 14a and 14b is provided in each
branch tube 7a, 7b respectively.

The two branch tubes 7a, 7b can be used simultaneously
to infuse liquid into two mammary veins of a collapsed
cow suffering from coli-mastitis.  Alternatively, tube
7a can be closed by tap 14a and tube 7b can be used for
infusion into a single vein, such as the jugular vein
of a diarrhoeic calf.

CLAIMS

1)    An infusion device comprising liquid supply means, a delivery tube and a injection means, the delivery tube interconnecting said supply means with said injection means and being arranged such that it presents an enlarged surface area along a portion of its length whereby said portion acts as heat receiving means.

2)    A device according to claim 1 wherein the heat receiving portion of the delivery tube is formed into a coil.

3)    A device according to claim 2 wherein successive turns of the coil are held in close relation to one another by restraining loops.

4)    A device according to claim 2 wherein successive turns of the coil are heat sealed or welded together.

5)    A device according to claim 2 wherein the coil is enclosed in a shrink-wrap membrane to hold successive turns of the coil in near relation to one another.

6)    A device according to any one of the preceding claims wherein the liquid supply means comprises a reservoir and control means for supplying a monitored amount of liquid from said reservoir into the delivery tube.

7)    A device according to claim 6 wherein the said
control means comprises a drip chamber and a control
valve.

8)    A device according to claim 6 or claim 7 which
further comprises a peristaltic pump arranged to pump
liquid from the said reservoir to the delivery tube.

9)    A device according to any one of the preceding
claims wherein the injection means comprises a hollow
needle or cannula.

10)  A delivery tube for use in the device according to
claim 1.

Fig.1

Fig.2